# EUROPEAN PATENT APPLICATION

(11) **EP 3 851 090 A1**
(43) Date of publication of application: **21.07.2021**
(21) Application number: 21150070.7
(22) Date of filing: 04.01.2021
(51) Int. Cl.: A61J 15/00

(54) **DEVICE FOR FIXING A NASOGASTRIC TUBE**

(30) Priority: 20.01.2020 IT 202000000955
(71) Applicant: Universita' Cattolica Del Sacro Cuore, 20123 Milano Mi (IT); Fondazione Policlinico Universitario Agostino Gemelli IRCCS, 00168 Roma (RM) (IT)
(72) Inventor: PELO, Sandro, 00168 Roma (IT); MORO, Alessandro, 00168 Roma (IT); GASPARINI, Giulio, 00168 Roma (IT); SAPONARO, Gianmarco, 00168 Roma (IT); FORESTA, Enrico, 00168 Roma (IT); BONIELLO, Roberto, 00168 Roma (IT); AZZUNI, Camillo, 00168 Roma (IT)
(74) Representative: Romano, Giuseppe

(57) **Abstract**

The present invention relates to a device for fixing and holding in place a nasogastric tube, comprising a needle-shaped element suitable to be inserted passing through the patient's columella or nasal septum; a ring connected or connectable to said needle-shaped element; and a fastening element connected or connectable to said ring and to said tube, in such a way as to retain the tube near said ring. The present invention allows to improve the stability of the tube, to dampen the small motions thereof and to avoid irritations from rubbing or contact, by reducing the displacement of the tube itself even in uncooperative patients who require protracted periods of enteral nutrition.

## Description

The present invention relates to a device for fixing and holding in place a nasogastric tube in patients subjected to enteral nutrition.

### Background

Patients under particular conditions, such as unconscious state or postoperative hospitalization after a surgical operation at the level of the head-neck district often require protracted periods of enteral nutrition.

Enteral nutrition is performed in most cases through the use of the nasogastric tube which allows the administered nutrients to reach directly the stomach, by avoiding the passage through the oral cavity and oesophagus and, then, not requiring deglutition. The nasogastric tube is inserted inside one of the nostrils and it is pushed as far as the gastric cavity; it is then fixed and held in place mainly by means of stitches made at the level of the nasal septum or the columella, or by means of positioning adhesive devices at the level of the nasal pyramid.

The displacement of the nasogastric tube seat is a frequent event, especially in case of long periods of enteral nutrition and above all it is caused by even unvoluntary motions of the patient.

Over the years, different methods and devices for fixing and holding in place the nasogastric tube have been proposed.

A first method provides to fix the nasogastric tube at the level of the nasal septum or of the columella by means of not re-absorbable stitches. In the specific case, the stitches are positioned at the level of the nasal septum or columella and subsequently knotted around the tube portion outgoing from the nostril. Such method is made easier by the addition of an eyelet at the tube level for the passage and anchoring of the stitch.

Another method widely used for fixing the nasogastric tube consists in applying skin patches on the nasal pyramid. Such patches are provided with a distal end placed so as to wind the tube portion outgoing from the nostril. Some devices make use of ergonomic adhesives to be applied at the level of the nasal pyramid and constitute an '"evolution" with respect to the classical devices for fixing by means of patches. An example is the device described in the patent US4140304; such device has an adhesive ergonomic portion which is placed on the nasal septum, connected to a flexible portion ending with a ring-like clip for fixing the nasogastric tube.

An additional widely used method provides the fixing of the nasogastric tube to one nostril by means of the use of pincers or hooks. In particular, the patent TW201415436 describes a device which is applied at the level of the nasal columella and which has laterally a ring-like clip for fixing the nasogastric tube passing through the nostril.

Another method, widely used too, provides to fix the nasogastric tube to the nasal septum. In this method, a catheter is made to pass around the nasal septum, with or without the use of magnets, and subsequently knotted and connected to the nasogastric tube, by means, for example, a ring-like clip, such as in case of the device described in the patent CN206603940.

Notwithstanding the variety of inventions in the field of interest, the fact of fixing and holding in place the nasogastric tube represents a still open problem, since, very often, the patients requiring enteral nutrition with nasogastric tube have poor levels of collaboration and/or poor level of consciousness and, then, voluntary or unvoluntary, they can cause the displacement of the nasogastric tube. Moreover, the up-to-now known devices and methods for fixing a nasogastric tube frequently, if used for long periods, result in irritations caused by friction or contact; this reduces the patient's comfort and, consequently, compliance. It follows that often the choice of the device for fixing and holding in place the tube has to be evaluated in a customized way, that is by keeping into consideration the compliance of the patient to be treated. Then, there is the need for arranging new and improved devices capable of fixing a nasogastric tube for long periods of enteral nutrition with stability and without inducing irritations by friction or contact, with the purpose of increasing the general compliance and, consequently, by reducing the incidence of both voluntary and unvoluntary displacements of the nasogastric tube.

### Technical problem solved by the invention

The object of the present invention is to solve the problems left open by the known art, by providing a device for fixing a nasogastric tube as defined in claim 1.

Additional features of the present invention are defined in the corresponding depending claims.

The advantages, together with the features and to the use modes of the present invention, will result evident from the following detailed description of preferred embodiments thereof, shown by way of example and not for limitative purposes.

### Brief description of figures

Hereinafter in this description reference will be made to the drawings reported in the enclosed figures, wherein:
- figure 1 shows a specific embodiment of the device for fixing a nasogastric tube to the columella, according to the present invention;
- figures 2A to 2D show a needle-shaped element passing through the hole performed at the level of columella and connected to the ring 13;
- figure 3 shows the ring connected to a fastening element;
- figure 4 shows in details the fastening element constituted by elastic material, provided with adjustable locking mechanism;
- figures 5A to 5D show examples of rings and needle-shaped elements according to the present invention.

### Detailed description of preferred embodiments

The present invention will be described hereinafter by making reference to the above-mentioned figures.

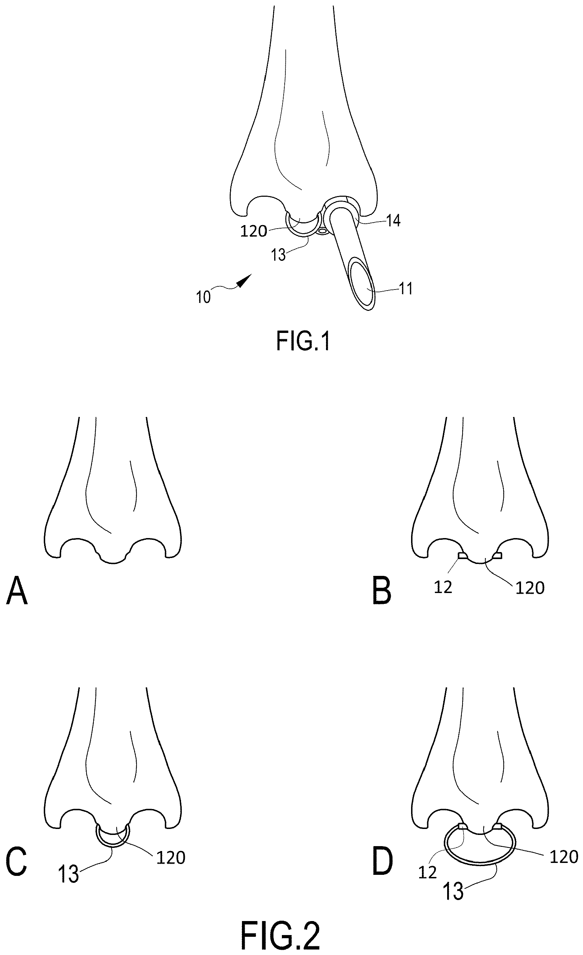

Figure 1 shows an example of device 10 for fixing a nasogastric tube 11 positioned at the level of the nasal columella.

The positioning of the device 10 takes place through perforation of columella or nasal septum 120. The perforation can be performed through the most common systems for performing piercing, that is by using for example a sterile needle with variable size.

In the device 10, the needle-shaped element 12, shown in detail in figure 2B, crosses the performed hole and connects to the ring 13 which in turn is connected to the fastening element 14. The fastening element 14 is a thread or a tape apt to fix the tube 11 near the ring 13 and, then, near the nasal columella.

The device 10 then allows to anchor with stability the nasogastric tube 11 even in uncooperative patients, by favouring its keeping in place. Moreover, the reduction in the direct contact between the tube and the columella or the nasal wall disadvantages the appearance of phenomena such as, otherwise frequent, irritations by contact.

The device 10 can further be used to fix any other tube, catheter or device passing through the nostril. Based upon the type of tube, catheter or device to be fixed, it is possible to select both the sizes of the hole and the materials and the sizes of the various components of the device 10, that is of the needle-shaped element 12, of the ring 13 and of the fastening element 14.

As shown in the specific embodiment shown in figure 2C, the needle-shaped element 12 and the ring 13 can be integrated with each other. In this case, the positioning of the element 12 at the level of the columella or of the nasal septum 120 corresponds to the positioning of the ring 13 itself.

In other embodiments, instead, the element 12 and the ring 13 can be connected by means of the reversible mutual connection means. In particular, such means, by way of example, can be pins, hooks, clips or holes, arranged in a compatible way therebetween on at least an end of the element 12 and at least an end of the ring 13.

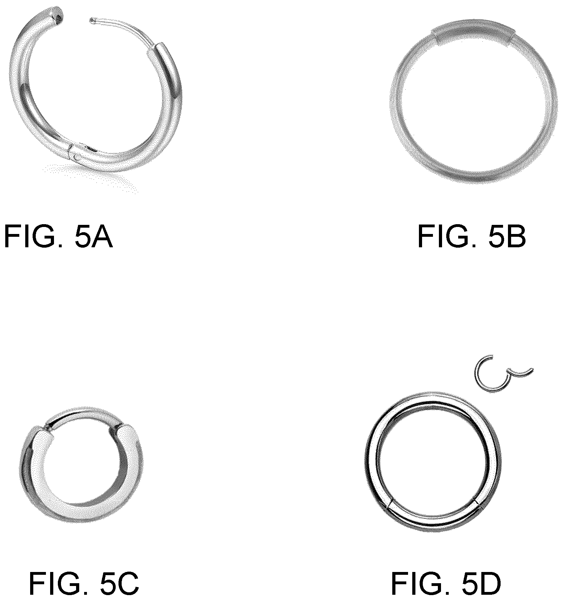

In the specific embodiment shown in figure 2D, the ring 13 is connected to the needle-shaped element 12 by means of a system of hooks and holes positioned at the two ends of the needle-shaped element 12 and of the ring 13. It is to be meant that any other system could advantageously be used which succeeds in hooking the ring 13, similarly to what takes place for the traditional piercings, for example - such for example illustrated in figures 5A to 5D - systems wherein the needle is hinged to the ring at one end or systems wherein the needle is sliding inside the ring, or systems wherein the needle is connected to the ring through screw elements, or systems wherein the needle is connected by interlocking on the ring through hooks and holes made at the ends of needle and ring. These and other modes for connecting the needle-shaped element to the ring are to be meant within the comprehension of a person skilled in the art and then it is not considered necessary to provide a more detailed description thereof.

The needle-shaped element 12 can consist of natural and/or synthetic polymers, even in combination therebetween, or of one or more metals or metal alloys. In the same embodiment it is possible that the material the needle-shaped element 12 is made of is identical to the one thereof the ring 13 is made.

The ring 13, in fact, preferably consists of one or more natural and/or synthetic polymers, even in combination therebetween.

In some embodiments, the ring 13 for example consists of plastics, latex or silicon. The use of gummy and/or elastic materials allows to obtain a highly flexible ring 13, capable of dampening the small motions of the nasogastric tube 11.

All components of the device 10, that is the needle-shaped element 12, the ring 13 and the fastening element 14, can be based upon hypoallergenic and dermo-compatible materials. Moreover, the selection of the material can be evaluated case by case, by keeping into consideration even possible allergies therefrom the patient suffers.

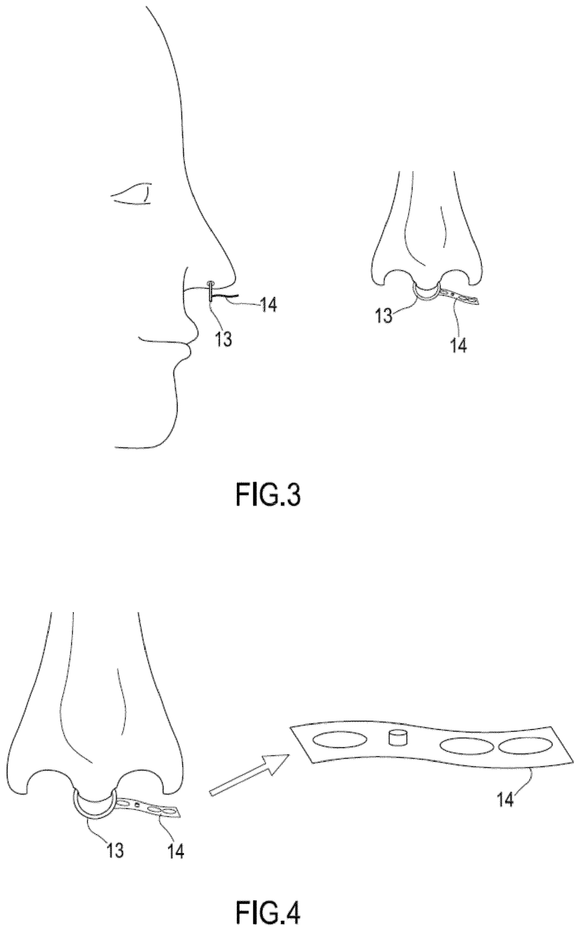

Figure 3 shows in detail the ring 13 and the fastening element 14. The element 14 is a thread or a tape made of natural and/or synthetic fibers connecting the ring 13 to the tube 11.

In some embodiments, the ring 13 and the fastening element 14 are integrated with each other, then the positioning of the ring 13 corresponds to the positioning of the fastening element 14; whereas, in other embodiments, the element 14 is wound around the ring 13.

In absence of an adjustable locking system, the fixing of the tube 11 to the ring 13 takes place by forming one or more knots.

The fastening element 14 can even be a suture thread. In some embodiments the fastening element 14 instead preferably consists of elastic material.

As shown in figure 4, the fastening element 14 can further include an adjustable locking mechanism. The adjusting locking mechanism generally is a mechanism which exploits the presence of holes, hooks or clips, positioned on the fastening element itself, with the purpose of adjusting the distance between the tube 11 and the ring 13 of the device 10.

In the specific embodiment shown in figure 4, the fastening element 14 is integral part of the ring 13 and it is an elastic band provided with an adjustable locking mechanism to adhere around the tube 11 and to fix it at the ring 13.

By using this mechanism, it is then possible to adjust in any moment the distance between the tube 11 and the ring 13.

The device 10 for fixing a nasogastric tube 11 described in the present invention, allows to reduce the displacement of the tube 11 even in uncooperative patients. The device 10 in fact anchors very firmly the tube to the columella or to the nasal septum 120 by means of a needle-shaped element 12, a ring 13 and a fastening device 14. Moreover, the indirect connection between the tube 11 and the columella or the nasal septum allows to dampen the motions of the tube, to reduce the irritations by contact and thus to increase the patient's comfort. Based upon what described, the device 10 could be widely used in case of patients which require protracted periods of enteral nutrition.

The present invention has been described sofar with reference to its preferred embodiments. It is to be meant that each one of the technical solutions implemented in the preferred embodiments, herein described by way of example, can advantageously be combined, differently from what described, with the other ones, to create additional embodiments, belonging to the same inventive core and however all within the protective scope of the herebelow reported claims.

## Claims

1. A device (10) for fixing a nasogastric tube (11) to a patient's nose, comprising:
• a needle-shaped element (12) suitable to be inserted passing through the patient's columella or nasal septum (120);
• a ring (13) connected or connectable to said needle-shaped element (12); and
• a fastening element (14) connected or connectable to said ring (13) and to said tube (11), in such a way as to retain the tube (11) near said ring (13).

2. The device according to claim 1, wherein said needle-shaped element (12) and said ring (13) comprise reversible mutual connection means.

3. The device according to claim 2, wherein said reversible mutual connection means comprises a system of hooks and holes positioned at the two ends of the needle-shaped element (12) and of the ring (13).

4. The device according to claim 1, wherein said needle-shaped element (12) and said ring (13) are integrated with each other.

5. The device according to any one of the preceding claims, wherein said ring (13) is made of plastic, latex or silicone.

6. The device according to any one of the preceding claims, wherein said fastening element (14) is integrally connected to said ring (13).

7. The device according to any one of the preceding claims, wherein said fastening element (14) is a thread or a tape made of natural and/or synthetic fibers.

8. The device according to any one of the preceding claims, wherein said fastening element (14) is made of elastic material.

9. The device according to claim 6, wherein said fastening element (14) is an elastic band and comprises an adjustable locking mechanism for adhering around said nasogastric tube, by fixing it at the ring.
